# EUROPEAN PATENT APPLICATION

(11) **EP 0 534 619 A2**
(43) Date of publication of application: **31.03.1993**
(21) Application number: 92307795.2
(22) Date of filing: 26.08.1992
(51) Int. Cl.: C12N 15/64, C12N 15/85, G01N 33/53, C12N 9/12

(54) **Expression cloning method**

(30) Priority: 26.08.1991 US 749545
(71) Applicant: RHODE ISLAND HOSPITAL, Providence, Rhode Island 02903 (US)
(72) Inventor: Sang, Junsheng, Providence, Rhode Island 02904 (US); Thompson, Nancy L., Barrington, Rhode Island 02806 (US); Hixson, Douglas C., Barrington, Rhode Island 02806 (US)
(74) Representative: Deans, Michael John Percy

(57) **Abstract**

Disclosed is a process for cloning a nucleotide sequence that encodes a protein. The process includes the steps of: preparing a recombinant molecule containing the nucleotide sequence and a vector that includes an expression control element operatively attached at linkage sites to the nucleotide sequence; introducing the recombinant molecule into host cells derived from a multi-cellular organism; identifying, among the host cells into which the recombinant molecule is introduced, cells expressing the protein by a means that specifically detects the protein; attaching the cells expressing the protein to a solid surface; and recovering the recombinant molecule from the attached cells. The same process can also be used to test the specificity of an antibody in its binding to an antigen, when a DNA sequence encoding the antigen is available.

## Description

The present invention relates generally to the manipulation of genetic materials and, more specifically, to the methods and kits for cloning a DNA sequence and for screening antibodies.

One of the fundamental approaches in understanding human diseases with a known or suspected hereditary basis is to characterize the genes involved. Tremendous efforts have been made to identify and clone genes that relate to many diseases with a genetic basis, including cancer. Characterization of these genes not only aids in our understanding of the disease processes, it also facilitate development of new diagnostic and therapeutic modalities.

The task of cloning an unknown gene where antibody to the protein product of the gene is the only available "handle" is laborious and time consuming. Basically two approaches have been developed. One is to use the antibody to purify the protein which is then sequenced and to synthesize degenerate oligonucleotide mixtures based on that amino acid sequence. The oligonucleotides are used as probes to screen a phage cDNA or genomic library and isolate the target genetic material. Another approach employs an expression system to express protein products of a cDNA library including the target gene. Antibody is then used to identify the positive clones. A phage vector with a prokaryotic promoter is the classic example in which phage plaque replicas on a filter are stained with antibody and this filter is then used as a template to locate positive plaques from the bacterial culture plate. Phage DNA with the insert of interest is subsequently recovered.

A eukaryotic expression cloning system has been successfully set up in recent years. E.g., see Seed, Nature 329:840 [1987]. This system has been developed for cloning cell surface molecules and is particularly useful where antibodies are available that do not recognize proteins expressed in a procaryotic system, such as those whose primary epitopes include sugar moieties. A highly efficient vector called CDM8 containing a cytomegalvirus/human immunodeficiency virus ("CMV/HIV") promoter was constructed by Seed and coworkers. In addition, the simian virus 40 ("SV40") origin contained in this vector enables these episomes to replicate up to 10⁵ copies per cell when they are propagated in any SV40 T/t containing host cells like the COS monkey cell line.

The Seed cloning strategy includes the following steps: 1) introducing a cDNA library constructed in CDM8 into COS cells by DEAE-dextran transfection or protoplast fusion, 2) "panning" the positive cells by removing the monolayer of cells and exposing them to an antibody coated culture dish so that those cells expressing the correct cDNAs on their cell membrane are captured by the antibody on the dish, 3) recovering the plasmid DNA from the panned cells and using it to transform the bacterial host MC1061/p3 to propagate and amplify it. Since the panned cells are never 100% pure for the clone of interest, it is necessary to repeat the above process until the only plasmid recovered expresses the protein product recognized by the antibody.

To be able to clone a target gene which is not highly expressed in the cDNA library, a minimum of 10⁵ independent copies are required to assure one expressed target. The low efficiency inherent in procedures of cell panning and recovery of plasmid cDNA demands a large initial pool of the cDNA library. Also, transformation of plasmid back to bacteria is a low recovery process as well, with an estimated efficiency of about 1 in 1000 copies. Overall, though antibody panning avoids protein purification and sequencing, it is still labor intensive, time consuming and costly.

In general, the invention, in one aspect, features a process for expression cloning a nucleotide sequence that encodes a protein.

The expression cloning process includes the steps of: preparing a recombinant molecule containing the nucleotide sequence of interest and a vector that contains an expression control element operatively attached at linkage sites to said nucleotide sequence; introducing the recombinant molecule into host cells derived from multi-cellular organism; identifying, among the fixed host cells, cells expressing the protein by a means that specifically detects the protein; attaching the cells expressing the protein to a solid surface; and recovering the recombinant molecule from the attached cells.

What is meant by "identifying" in the present invention is the process of contacting the detecting means with the cells into which the recombinant molecule is introduced and does not necessarily denote locating the cells expressing the protein. The order in performing the identifying step and the attaching step may be reversed, if desired. Also, one may attach the host cells expressing the protein to a solid surface by centrifugation. Alternatively, one may achieve the same objective taking advantage of the fact that cells may adhere to the surface by themselves. The attachment may be on the bottom surface of a culture dish or a glass slide, either coated with a matrix or not (see below for details), or other suitable containers or support.

In one embodiment of the above process, the attached cells are fixed before the identifying step (i.e., to render the cellular membranes semipermeable), thereby allowing influx of the detectable means into the fixed host cells while preventing exit of the recombinant molecule and the protein. The fixation can be effected by any suitable methods, such as using a chemical reagent. Preferably, acetone is used as the fixative.

It is also preferable that in the above process the detecting means is an antibody or a ligand that specifically binds to the protein and is linked to a fluorescent group, an enzyme, or a radioactive isotope.

In another preferred embodiment of the above process, the host cells into which the recombinant molecule is introduced are cultured on a matrix, such as collagen coated on a culture dish, before the identifying step.

In the above process, it is desirable that the nucleotide sequence in the recovered recombinant molecule may be enriched. The enrichment may be achieved by PCR amplification, using a pair of vector primers that flank the linkage sites between the nucleotide sequence and the vector.

Preferably, the recombinant molecule prepared in the above process is capable of replication in the host cells. The expression vector CDM8 can be used to construct such a recombinant molecule. It is also preferable that the host cells used in the process be COS cells.

The protein to be expressed in the host cells may be post-translationaly modified, such as a glycosylated protein.

In another aspect, the invention features a method of testing specificity of an antibody in its binding to a protein, where a nucleotide sequence coding for that protein is available. The testing method includes the steps of: preparing a recombinant molecule that contains that nucleotide sequence and a vector that contains an expression control element operatively attached at linkage sites to the nucleotide sequence; introducing the recombinant molecule into host cells derived from a multi-cellular organism; and detecting, among the host cells into which the recombinant molecule is introduced, cells expressing the protein with the antibody which is detectably labeled to determine specificity of the antibody in its binding to the protein. Note that if necessary and where appropriate, the host cells into which the recombinant molecule is introduced may be fixed before the identifying step in a manner set forth above (see the text regarding the expression cloning process of the invention) as to enable influx of the detectable means into the host cells. Also, the antigen protein to which the specific binding of an antibody is tested may be a protein post-translationaly modified (e.g., glycosylated) by the host cells.

Still within the invention, in a further alternative aspect thereof, is a kit for expression cloning a nucleotide sequence or for testing the binding specificity of an antibody to a protein encoded by the nucleotide sequence. The kit provides the following items: a vector that includes an expression control element; an adaptor compatible to the vector; a pair of vector primers; a container, for example a culture dish either coated on its interior bottom surface with a matrix or without any coating; a cell fixative; and an antibody to which an enzyme, a fluorescent group, or an isotope is linked. Note that the antibody serves as a secondary antibody in an immunoassay. As mentioned above, the fixative may be used to treat cells, thereby rendering their membranes permeable to a detectable means which is made of the antibody that is also provided in the kit.

If necessary, the above-described kit may further include two more items: digestion buffer (see below for an exemplary composition) that can be used for extraction of plasmid from plasmid-containing cells; and a means for removing or isolating a portion of the matrix coating from the reminder thereof. As an example, a gauge needle, which can be used to remove any portion of a matrix, can be provided. Alternatively, a so-called "cloning ring" is provided so that an operator may initiate a DNA extraction process on cells grown on a specific area of the matrix only.

Other kit embodiments include an expression cloning kit which provides an expression vector; an adaptor compatible to the vector; a pair of vector primers; a culture dish either with a matrix coated on its interior bottom surface or without any coating; a cell fixative; an absorbent sheet onto which an enzyme substrate is attached; an antibody to which the enzyme is linked; a means for removing or isolating a portion of the substrate from the reminder thereof; and digestion buffer. Except for the enzyme-containing absorbent sheet, other items in the kit are similar to those described above.

The term "vector" used herein refers to a small, circular, extrachromosomal DNA molecule capable of being introduced into a host cell, and the term "expression vector" refers to a vector which contains at least one expression control element (such as a promotor, or a promotor with an enhancer or a splice and polyadenylation signal) and is capable of expressing a nucleotide sequence operatively linked to it. In other words, "operative linkage" means that an expression vector and an encoding DNA sequence are so joined that transcription of the sequence is possible due to the function of at least one expression control element. The two points where the insert and the vector are joined are called "linkage sites".

The term "nucleotide sequence" is meant to include any DNA fragments prepared by whatever available and suitable methods and not to be limited to those of a cDNA library. The term "nucleotide sequence that encodes a protein" includes both translated and non-translated regions.

What are meant by "vector primers" are a pair of oligonucleotides that correspond to the vector sequences flanking the linkage sites between an expression vector and a DNA sequence and can be used as primers in enrichment of the DNA sequence insert, such as PCR amplification.

Other terms used will be defined or explained as they appear below.

According to the present cloning method, the cells to be recovered are positively identified before recovery, so that recovery is very efficient. Further, when PCR amplification is used to enrich the desired DNA sequence in the recovered cells, the recovery of the target sequence is only limited by the DNA extraction yield (which is high according to the invention) and performance of the PCR method. Moreover, when transfected cells are fixed, one can use the cloning method to isolate a DNA sequence that encodes an intracellular protein.

Other features and advantages will be apparent from the following description of the preferred embodiments, and from the claims.

The drawings are first described.

Fig. 1 is a diagram of the procedures followed in one embodiment of this invention.

Fig. 2 is a representation of a vector, pCDM8, used in preparing a construct containing a Cell-CAM 105LF cDNA ("Cell-CAM 105LF construct" or "Cell-CAM 105LF plasmid").

Fig. 3A is a representation of a construct containing Cell-CAM 105LF cDNA, showing two restriction sites and three binding sites for PCR primers Hx and Np.

Fig. 3B is a representation of two PCR products from amplification using Cell-CAM 105LF construct as the template and Hx and Np as the primers.

Fig. 4 is two photographs showing results from immunofluorescence assays of transfected cells. (Upper: fluorescence microscopy; lower: phase microscopy.)

Fig. 5 is two photographs showing results from immunofluorescense assays of untransfected cells. (Upper: fluorescence microscopy; lower: phase microscopy.)

Fig. 6A is a photograph of agarose gel electrophoresis (stained by ethydium bromide), showing PCR products of vector-primed insert amplification of DNA from positive cells.

Fig. 6B is an autoradiograph of Southern blot analysis from the agarose gel of Fig. 6A.

We have developed an expressing cloning method in which transfected cells are attached to the surface of a container (e.g., a culture dish or a glass slide) prior to the stage of recovering positively identified cells (i.e., cells containing the target DNA sequence) for extraction of the desired recombinant molecules in them. Our method can be employed to clone a nucleotide sequence encoding a cell surface protein, a secreted protein, or an intracellular protein. When a sequence coding for an intracellular protein is to be cloned, the cellular membranes of the transfected cells are rendered semipermeable, such as by acetone fixation, so that a detecting group (e.g., such as a fluoresently labeled antibody against the expressed protein) may enter the cells, while plasmids and the expressed protein remain within the cell.

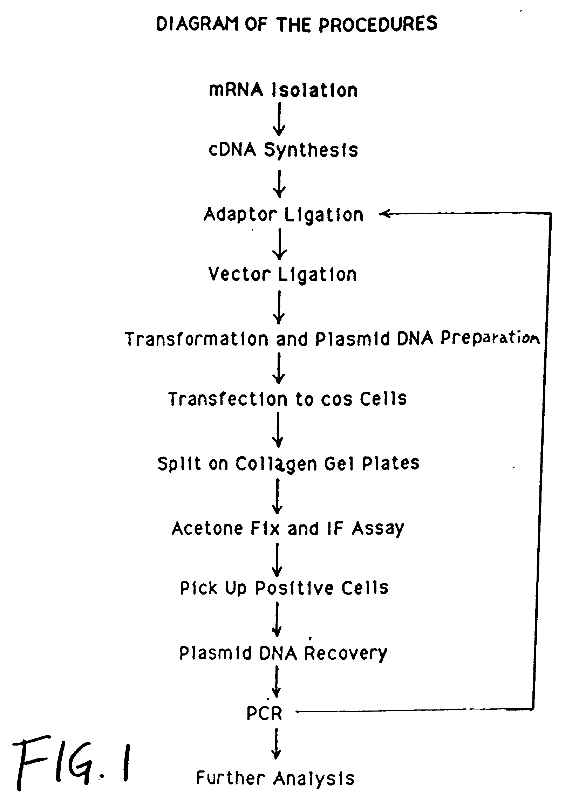

For illustration purposes, a diagram of cloning procedures representing one embodiment of the invention is shown in Fig. 1. More details of the cloning procedures are set forth below.

### 1. Constructions of cDNA library

Poly A RNAs (mRNAs) are isolated from the target source (tissue or cultured cells) which is identified as containing the mRNA species of interest or its encoded protein by available detecting methods, such as hybridization or antibody binding. cDNAs are then generated from the isolated mRNAs by reverse transcriptase using conventional methodology. As a general rule, 1-5 µg mRNA are used as template to establish a representative cDNA library.

### 2. Ligation to expression vector

cDNAs are then ligated to an expression vector, such as the pCDM8 vector developed by Seed et al. (Seed, Nature 329:840 [1987], herein incorporated by reference). In order to increase efficiency of our cloning method, a vector with both high replication and expression rates is preferred.

Because of the concern over ligation efficiency, designed adaptors (short oligonucleotides) containing specific restriction sites can be ligated to cDNAs first, then to the expression vector which has compatible restriction sites. For example, a BstX I adaptor and the pCDM8 expression vector are compatible with each other and can be used for this purpose. These materials are commercially available from Invitrogen, Inc., San Diego, CA. For increasing the ratio of target sequences in the library, construction of sub-libraries may be desired based on predicted size range of the corresponding messenger RNA. This can be achieved by fractionating cDNA on an agarose gel, then recovering cDNAs from an excised gel slice based on size location.

### 3. DNA transfer

The plasmid DNA of a cDNA library or sub-library is introduced into host cells by various techniques well known in the art, e.g., transfection. Transfection can be performed by means of electroporation, protoplast fusion, DEAE dextran, or calcium phosphate. The main criteria should include high efficiency of DNA transfer and that the cell viability after the transfer is maintained.

Host cells derived from a multi-cellular organism, such as a mammal, an insect, or a plant, are used in this invention. Preferably, the host cells do not express the type of protein encoded by the targeted DNA sequence. However, such expression is nonetheless tolerable (1) if the antibody used to identify positive cells only binds to the recombinant protein (i.e., protein encoded by the insert in the recombinant molecule) and not to the native, host protein, or (2) if the expression level of the host protein is far lower than that of the recombinant protein.

It is preferable that the culture dish be coated with collagen. Such a coating enables one to conveniently remove positive cells from the dish for further treatment. Collagen is preferred because it serves as an excellent matrix for cell growth. Also, because of its viscosity, it is easy to pick up with a needle or the like and thereby facilitates recovery of cells attached or adhered onto it. Further, collagen does not interfere with immunoassay which is used to identify cells containing the target DNA sequence. Other substances which can also be used as a matrix for cell growth include agarose.

Alternatively, transfected cells can be plated in multi-well plates if enzyme-linked immunosorbent assay ("ELISA") is to be used to identify cells of interest.

### 4. Identification of positive cells

Preferably, especially when DNA sequence encoding an intracellular protein is to be cloned, the transfected cells growing on dishes are fixed first by treatment with a suitable fixative, e.g., immersed in cold acetone for 10 minutes and then allowed to air dry. Suitable chemical reagents, are formaldehyde, ethanol, glyceraldehyde, or formalin. Two of the most important criteria in selecting a fixative are that it does not destroy epitopes of the expressed antigen protein, and that it does not interfere with the identification of positive cells and recovery of the DNA introduced into the cells.

Indirect immunofluorescence ("IF") can be used to identify positive cells, which involves the steps of blocking with normal serum of the same species as the second antibody reagent, and incubation first with unlabeled primary antibody directed toward the specific antigen and then the second antibody (directed against the species and immunoglobulin class of the first antibody) conjugated to fluorescent tag (eg., FITC). Alternatively, one may employ the avidin-biotin-fluoresence method. See Harlow, E. and Lane, D., Antibodies, Cold Spring Harbor Lab Press, 1988, herein incorporated by reference. Antibody-reactive cells can be identified by observation on a fluorescence microscope and the area with the positively identified cells is carefully marked on the bottom of the culture dish.

As mentioned above, ELISA can also be used to identify positive cells either manually or using an automated plate reader (a type of spectrophotometer). A similar enzyme-linked assay can be used by the use of an absorbent sheet, such as a nitrocellulose sheet or a filter paper, that is pre-soaked or coated with an enzyme substrate. Such a sheet is stained when placed against the cells. The stained replica sheet can then be used locate the target cells. This method is most suitable for cloning DNA sequences encoding secreted proteins. Enzymes suitable for enzyme-linked assays include alkaline phosphatase, glucose oxidase and luciferase.

Isotopes, such as ³²P, can also be used to detect positive cells. Because of its high sensitivity, this detection method is especially suitable when the recombinant molecules have a low replication or expression level.

### 5. Recovery of the plasmid DNA from cells

Where the transfected cells are cultured on a collagen gel or matrix, the positively identified cells can be picked up by using a fine gauge hypodermic needle to score the collagen layer and collect the marked piece of collagen gel containing such cells. The collagen fragment typically contains 50-100 cells and is transferred to a sterile microfuge tube. 10 ul of digestion buffer (i.e., a solution used to extract DNA from cells; for example, 50 mM Tris, 1 mM EDTA, 0.5% Tween 20 and 200 ng/ml proteinase K) is added to the tube which is then incubated at 55°C for 3 hours to release DNA from the cells. After a brief centrifugation, the supernatant containing plasmid DNA can be subjected to further treatment.

There are other methods to pick up positive cells. For example, one can place a tiny piece of filter paper pre-soaked with a trypsin solution against the positive cells. Once trypsinized, the cells can be picked up by the paper readily for extraction of DNA.

Alternatively, one can treat cells with digestion buffer while cells remain in a dish or a multi-well plate. In the former situation, a cell culture cloning ring (Bellco Glass, Vincland, NJ), which is a small glass cylinder open at both ends, can be used. More specifically, the cloning ring is placed on the dish so that the desired cell is inside, then the digestion buffer is placed in this isolated area, and finally the mixture is transferred out of the ring and into a tube for further treatment.

### 6. Amplification of DNA insert

Plasmid DNA from positively identified cells can be enriched by any suitable methods. For example, one can adopt the conventional approach, i.e., transforming bacterial host cells with the plasmid DNA, followed by multiplication of the transformed cells and isolation of the plasmid DNA therefrom.

Preferably, PCR, an enzymatic amplification method, can be employed to specifically enrich the DNA insert, using as primers a pair of oligonucleotides corresponding to vector sequences flanking the cloning sites.

When the PCR method is used, the criteria for selection of oligonucleotides as primers include: (1) having a G-C content of about 50% and a size of 18-30 base pairs, (2) as proximate to the cloning sites as possible, and (3) preserving desirable restriction sites in the vector.

Also, when more than one PCR product is detected, which is an indication that either a single cDNA has been cloned and at least one of the primers hybridizes to regions other than those selected, or that two or more cDNAs have been picked up. In the former situation, a different pair of primers can be used and tested, if necessary or desired. In the latter case, i.e., when DNA fragments obtained are not a single population, those fragments can be ligated back to the vector and steps 3-6 above will be repeated to further enrich and isolate the cDNA clone.

Furthermore, when any extra base pairs added to the 5' end (as a result of repeated PCR amplification and ligation) affect the expression of the target gene, the adaptors can be modified to incorporate restriction sites which will enables the additional bases to be digested off and permit unidirectional orientation of amplified inserts with respect to the vector.

Another enzymatic amplification method, the so-called "3SR (self-sustained sequence replication) system, can also be used, if desired. A reagent kit for the 3SR system can be obtained from Bartels Diagnostics, Issaquh, WA.

### CLONING OF A CELL-CAM 105LF cDNA INSERT

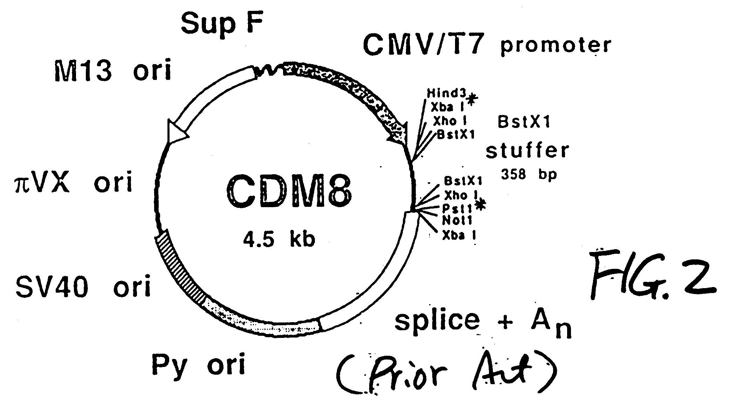

A DNA sequence encoding an Ecto-ATPase, a.k.a. "Cell-CAM 105LF", has recently been isolated and characterized. See Lin et al., J. Biol. Chem. 264:14408 [1989]. Lin and coworkers used pCDM8 (Fig. 2) to prepare a cell-CAM 105LF construct (Fig. 3A) by inserting a cell-CAM 105LF cDNA (i.e., thickened line in Fig. 3A) into two restriction sites, Xba I and Pst I (each indicated by an asterisk in Fig. 2).

We used the Fig. 3A construct as the starting material to demonstrate the efficiency and sensitivity of our cloning method.

10 µg cell-CAM 105LF plasmid was used to transfect 5x10⁵ COS cells (American Type Culture Collection, Rockville, MD, Access No. 1650) by the LIPOFECTIN™-mediated method (GIBCO/BRL, Grand Island, NY) on a 60 mm dish for 5-8 hours, with an efficiency rate of as high as 5x10³ cells/µg plasmid. Cells were then fed with media containing 10% fetal bovine serum and incubated overnight. 12-24 hours later, cells were split 1:2 and plated on collagen coated dishes and incubated further for 24-36 hours. Collagen was prepared from rat tail tendons, which is known as "rat tail collagen". For preparation procedures, see Siraca et al., Proc. Natl. Acad. Sci. USA 74:283-287 [1977]. Alternatively, commercial preparations of collagen such as a bovine tendon collagen with a tradename of "Vitrogen" are available from suppliers including Flow Laboratories, McLean, VA.

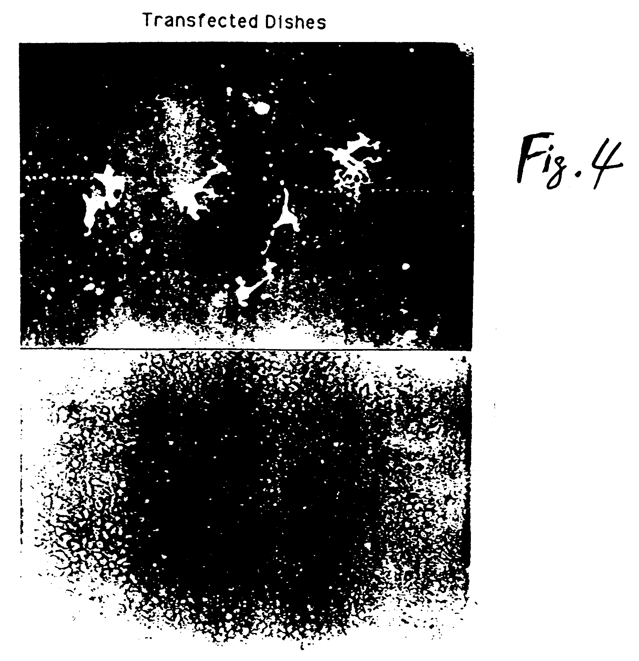

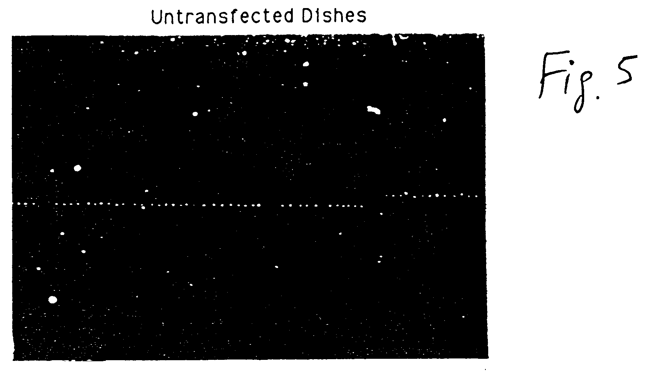

The transfected cells growing on collagen-coated dishes were fixed in cold acetone and then allowed to air dry before they were subjected to an indirect immnofluorescence assay. The monoclonal antibody used was generated in our laboratory (for procedures, see Mowery, et al., Hepatology 13:47 [1991], which is herein incorporated by reference). Secondary reagents were purchased from Vector Lab Inc. (Burlingame, CA). Recognition of single positive cells by the immunofluorescence staining was possible because each transfected copy of the plasmid could replicate up to 10⁵ copies in the host cell and each copy contained a strong promoter leading to a high level of expression of the inserted cDNA. Antibody-reactive cells were identified by observation on a fluorescence microscope (Fig. 4). As an control, untransfected cells were also fluorescently stained and microscopically observed (Fig. 5). The area corresponding to the positive cells was carefully marked on the bottom of the culture dish.

The positive cells were conveniently removed together with the matrix by means of a fine gauge hypodermic needle. The collagen fragment typically contained 50-100 cells and was transferred to a sterile microfuge tube. 10 µl of digestion buffer (see above for composition) was added to the tube which was then incubated at 55°C for 3 hours to release DNA from the cells. After a brief centrifugation, the supernatant containing plasmid DNA was used as a template for PCR amplification.

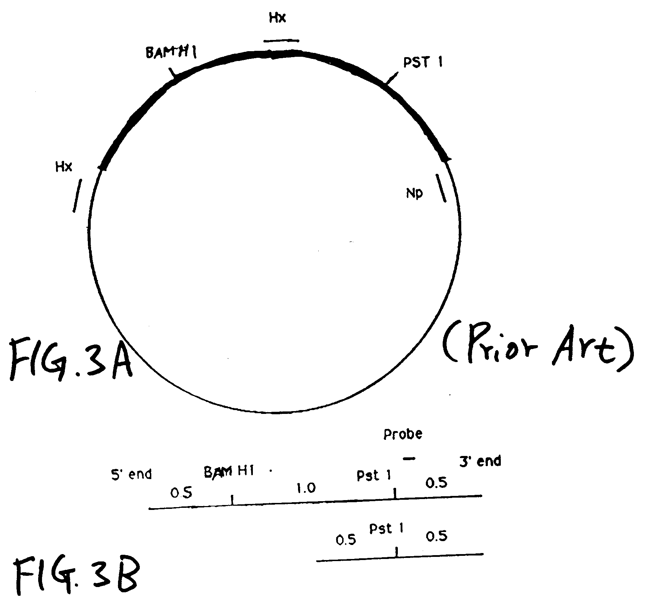

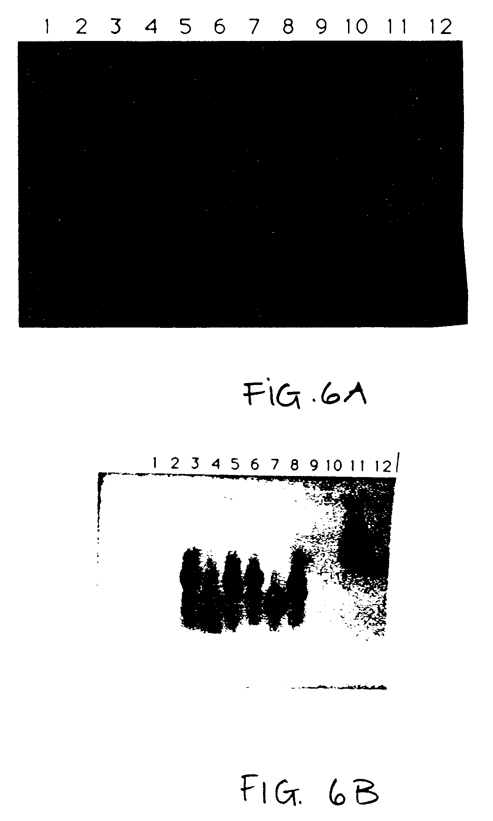

A pair of oligonucleotides, designated as Hx and Np by us, to serve as primers for PCR were designed based on regions of CDM8 vector sequences flanking the cloning sites. These primers are as follows (5' end to 3' end): 
+ strand ("Hx"):
CCCACTGCTTAACTGGCTTATCGA (SEQ ID NO:1)
- strand ("Np"):
GTCACACCACAGAAGTAAGGTTCC (SEQ ID NO:2)
PCR cycles were performed as follows: denaturation, 94°C, 1 min.; annealing, 65°C, 1 min.; and extension, 72°C, 3 min. The products of the PCR were analyzed by agarose gel electrophoresis and ethidium bromide staining (Fig. 6A), and subsequently by Southern blotting using a probe shown in Fig. 3B (Fig. 6B).

For Figs. 6A and 6B, samples applied to the agarose gel are as follows. Lanes: 1, DNA molecular weight markers; 2, PCR amplification products from CDM8; 3, PCR amplification products from Cell-CAM 105LF plasmid; 4, Pst I digestion of Lane 3 sample; 5, BamH I digestion of Lane 3 sample ; 6, PCR amplification products from DNA of picked positive cells; 7, Pst I digestion of Lane 6 sample; 8, BamH I digestion of Lane 6 sample; 9, "no template" control; 10, "untransfected cells" control; 11, cell-CAM 105LF plasmid; 12, PCR amplified λDNA. When PCR amplification was performed, primers Hx and Np were used except for Lane 2 where primers Hx and Ns (5' GCAGGCGCAGAACTGGTAGGTAT 3', SEQ ID NO: 3) were used instead.

As shown in Fig. 3A, primer Hx hybridizes to two regions, i.e., a sequence flanking one cloning site as designed and an unexpected sequence near the middle of the cell-CAM 105LF insert (positions 798-821 based on the numbering system of Lin et al., *Id*.). This explains why two bands were observed on Lanes 3 and 6, with the upper one corresponding to a PCR product encompassing the entire cDNA insert. Lanes 3-5 show patterns which were expected and identical to those of Lanes 6-8, indicating that the cDNA cloned by our method was indeed the cell-CAM 105LF insert.

We have thus demonstrated conclusively that our method is operative in expression cloning a cDNA.

### TESTING OF ANTIBODY SPECIFICITY

Our cloning method can also be adapted to test specificity of antibodies, e.g., monoclonal antibodies, in their binding to a protein antigen when a DNA sequence encoding that antigen is available. Such method is of great importance, in both scientific research and commercial applications, since it can be used for screening antibodies and selecting specific antibody at low costs even when the antigen is not available. The antibodies (e.g., monoclonal antibodies) to be screened can be generated by injecting into a proper animal crude protein preparation from cells transfected with an expression vector containing a DNA sequence coding for the antigen of interest.

The antibody testing or screening method is as follows. First, an expression vector containing the encoding DNA is introduced into suitable host cells. Preferably, the cellular membranes are rendered semipermeable using a suitable fixative, such as acetone if the expressed antigen is an intracellular protein.

The antibody to be tested, after being detectably labeled (e.g., directly or indirectly linked to a fluorescent compound, a radioactive isotope, or a proper enzyme), is then used to detect any expressed antigen protein. The presence of positive cells indicates that the antibody to be tested specifically binds to the expressed antigen.

### CLONING/ASSAY KITS

Kits for the above-described methods, i.e., expression cloning a DNA sequence and testing/selecting antibodies, can be provided for the convenience of those who want to practice the methods.

With such a cloning kit, a user who has access to a desired detecting antibody and to a suitable host cell line or strain, can readily start the task of cloning a nucleotide sequence that encodes a protein. By the same token, an antibody-testing kit enables one to screen for the desired antibody as soon as a DNA sequence encoding an antigen protein of interest is available.

Preparation of such kits can readily be achieved in the light of the above description by persons of ordinary skills in the art of preparing such hits. Also, use of such kits is apparent from description of the methods for cloning DNA sequence and for testing antibodies above.

SEQUENCE LISTING

INFORMATION FOR SEQUENCE IDENTIFICATION NUMBER: 1:

SEQUENCE CHARACTERISTICS:

LENGTH: 24
TYPE: nucleic acid
STRANDEDNESS: single
TOPOLOGY: linear

SEQUENCE DESCRIPTION: SEQ ID NO: 1:

CCCACTGCTT AACTGGCTTA TCGA 24

INFORMATION FOR SEQUENCE IDENTIFICATION NUMBER: 2:

SEQUENCE CHARACTERISTICS:

LENGTH: 24
TYPE: nucleic acid
STRANDEDNESS: single
TOPOLOGY: linear

SEQUENCE DESCRIPTION: SEQ ID NO: 2:

GTCACACCAC AGAAGTAAGG TTCC 24

INFORMATION FOR SEQUENCE IDENTIFICATION NUMBER: 3:

SEQUENCE CHARACTERISTICS:

LENGTH: 23
TYPE: nucleic acid
STRANDEDNESS: single
TOPOLOGY: linear

SEQUENCE DESCRIPTION: SEQ ID NO: 3:

GCAGGCGCAG AACTGGTAGG TAT 23

## Claims

1. A process for cloning a nucleotide sequence that encodes a protein, which process comprises the steps of:
preparing a recombinant molecule containing said nucleotide sequence and a vector that includes an expression control element operatively attached at linkage sites to said nucleotide sequence;
introducing said recombinant molecule into host cells derived from a multi-cellular organism;
identifying, among said host cells into which said recombinant molecule is introduced, cells expressing said protein by means that specifically detects said protein, said detecting means being an anitbody or a ligand that specifically binds to said protein and is linked to a fluorescent group, an enzyme, or a radioactive isotope;
attaching said cells expressing said protein to a solid surface; and
recovering said recombinant molecule from said attached cells.

2. The process of claim 1, which process further comprises a step of fixing said host cells into which said recombinant molecule is introduced before said identifying step, thereby allowing influx of said detecting means into said fixed host cells while preventing exit of said recombinant molecule and said protein.

3. The process of claim 2, wherein said fixing step is effected by treatment with a chemical reagent.

4. The process of claim 3, wherein said reagent is acetone.

5. The process of claim 1, wherein said host cells into which said recombinant molecule is introduced are cultured in a container the bottom face of which is coated with a matrix before said identifying step.

6. The process of claim 5, wherein said matrix is collagen.

7. The process of claim 1, which process further comprises a step of enriching said nucleotide sequence after the recovering step.

8. The process of claim 7, wherein said nucleotide sequence is enriched by enzymatic amplification with a pair of vector primers that flank said linkage sites between said nucleotide sequence and said vector.

9. The process of claim 8, wherein said enzymatic amplification is PCR amplification.

10. The process of claim 1, wherein said recombinant molecule is capable of replication in said host cells.

11. The process of claim 10, wherein said vector is pCDM8.

12. The process of claim 1, wherein said host cells are COS cells.

13. The process of claim 1, wherein said protein is post-translationally modified by said host cells.

14. The process of claim 13, wherein said post-translational modification is glycosylation.

15. The process of claim 1, wherein said multi-cellular organism is a mammal.

16. A process of claim 1, wherein said cells expressing said protein are identified first and, after said identification, are attached to said solid surface.

17. A process of claim 1, wherein said cells expressing said protein are attached to said solid surface first and, after said attachment, are identified by means that specifically detects said protein.

18. A process for testing specificity of an antibody in its binding to a protein, which process comprises the steps of:
preparing a recombinant molecule containing a nucleotide sequence that encodes said protein and a vector that includes an expression control element operatively attached to said nucleotide sequence;
introducing said recombinant molecule into host cells derived from a multi-cellular organism; and
detecting, among said host cells into which said recombinant molecule is introduced, cells expressing said protein with said antibody which is detectably labeled to determine specificity of said antibody in its binding to said protein.

19. The process of claim 18, which process further comprises a step of fixing said host cells into which said recombinant molecule is introduced before said identifying step, thereby allowing influx of said antibody into said fixed host cells.

20. The process of claim 19, wherein said cellular membranes are rendered semipermeable by treatment with a chemical reagent.

21. The process of claim 20, wherein said reagent is acetone.

22. The process of claim 21, wherein said protein is post-translationally modified.

23. The process of claim 22, wherein said post-translational modification is glycosylation.

24. The process of claim 18, wherein said recombinant molecule is capable of replication in said host cells.

25. The process of claim 24, wherein said vector is pCDM8.

26. A kit for expression cloning a nucleotide sequence or for testing the binding specificity of an antibody to a protein encoded by said nucleotide sequence, said kit comprising:
a vector that includes an expression control element;
an adaptor compatible to said vector;
a pair of vector primers;
a container;
a cell fixative; and
an antibody to which an enzyme, a fluorescent group, or an isotope is linked.

27. The kit of claim 26, wherein said container is coated with a matrix on its interior bottom surface.

28. The kit of claim 27, further comprising:
digestion buffer; and
means for removing or isolating a portion of said matrix coating from the remainder thereof.

29. An expression cloning kit comprising:
a vector that includes an expression control element;
an adaptor compatible to said vector;
a pair of vector primers;
a container;
a cell fixative;
an absorbent sheet onto which an enzyme substrate is attached;
an antibody to which said enzyme is linked;
means for removing or isolating a portion of said matrix from the remainder thereof; and
digestion buffer.

30. The kit of claim 29, wherein said container is coated with a matrix on its interior bottom surface.
